# EUROPÄISCHE PATENTSCHRIFT

(11) **EP 0 857 711 B1**
(45) Veröffentlichungstag und Bekanntmachung des Hinweises auf die Patenterteilung: **04.06.2003**
(21) Anmeldenummer: 98810044.2
(22) Anmeldetag: 27.01.1998
(51) Int. Cl.: C07C 43/295, C07C 49/84, A01N 31/16

(54) **Verfahren zur Herstellung von halogenierten Hydroxydiphenylverbindungen**
Process for the preparation of halogenated hydroxy diphenyl compounds
Procédé pour la préparation de composés hydroxy diphényls halogénés

(30) Priorität: 05.02.1997 EP 97810064
(43) Veröffentlichungstag der Anmeldung: 12.08.1998
(73) Patentinhaber: Ciba Specialty Chemicals Holding Inc., 4057 Basel (CH)
(72) Erfinder: Burckhardt, Urs, 4051 Basel (CH); Di Teodoro, Armando, 79618 Rheinfelden (DE); Hölzl, Werner, 68440 Eschentzwiller (FR); Reinehr, Dieter, 79400 Kandern (DE); Zink, Rudolf, 4106 Therwil (CH); Sauter, Hanspeter, 79650 Schopfheim (DE); Gronde, Uwe, 79618 Rheinfelden (DE)

(56) Entgegenhaltungen:
- EP-A- 0 384 043
- DE-A- 1 493 776
- D. C. ATKINSON ET AL: "Substituted (2-phenoxyphenyl)acetic acids with antiinflammatory activity" JOURNAL OF MEDICINAL CHEMISTRY, Bd. 26, Nr. 10, Oktober 1983, WASHINGTON US, Seiten 1353-1360, XP002034909
- "The Merck Index, Twelfth Edition" 1996 , MERCK & CO. , WHITEHOUSE STATION, NJ XP002064483 * Seite 1646, Verbindung Nr. 9790 *

## Beschreibung

Die vorliegende Erfindung betrifft die Herstellung von halogenierten Hydroxydiphenylverbindungen der Formel 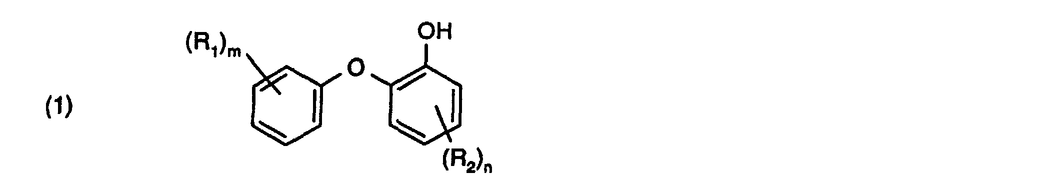 worin
R₁ und R₂Cl;
m 2; und
n 1 oder;
m und n 1;
bedeuten. sowie die Verwendung dieser Verbindungen als Desinfektionsmittel zum Schützen von organischen Materialien vor Mikroorganismen.

Die Herstellung von halogenierten Hydroxydiphenylverbindungen, insbesondere von 2-Hydroxy-2',4,4'-trichlorodiphenylether (Triclosan; Verbindung der Formel (3)) erfolgt gewöhnlich durch Diazotierung und anschliessende Hydrolyse von 2-Amino-2',4,4'-trichlorodiphenylether (TADE; Verbindung der Formel (2)).

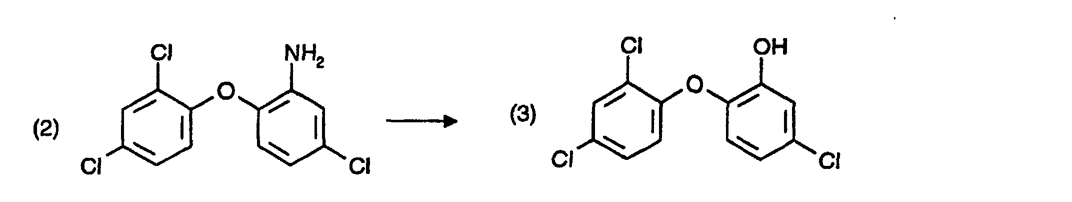

Allerdings ist die Ausbeute bei dieser Herstellungsmethode unbefriedigend, da verschiedene chemische Konkurrenzreaktionen stattfinden können.

Im Journal of medicinal Chemistry (1983), 1354-1360 wird die Herstellung von 2-Phenoxyphenyl)-essigsäuren beschrieben durch Acylierung eines Phenolats, Veretherung zur Phenoxyphenylacylverbindung und anschliessender Oxidation zur entsprechenden Phenoxyphenylessigsäure.

In der EP-A-0,384,043 wird ein Verfahren zur Herstellung von bromierten Diphenyletherverbindungen offenbart. Dabei weird eine substituierte Phenolverbindung mit einer halogenierten Phenylcarbonylverbindung im alkalischen Medium umgesetzt. Die Base wird dabei in äquimolaren Mengen oder im Überschuss, bezogen auf die eingesetzte Phenolverbindung, eingesetzt.

Der vorliegenden Erfindung liegt daher die Aufgabe zugrunde, ein wirtschaftliches Verfahren zur Herstellung von Halogen-hydroxydiphenylverbindungen zu finden, bei dem unerwünschte Nebenreaktionen unterdrückt werden.

Die Aufgabe wird erfindungsgemäss gelöst durch eine vierstufige Reaktion, worin in der ersten Stufe eine halogenierte Benzolverbindung acyliert, in der zweiten Stufe die acylierte Verbindung mit einer halogenierten Phenolverbindung verethert, in einer dritten Stufe die veretherte Verbindung oxidiert und in einer vierten Stufe die oxidierte Verbindung hydrolysiert wird, entsprechend dem folgenden Reaktionsschema: 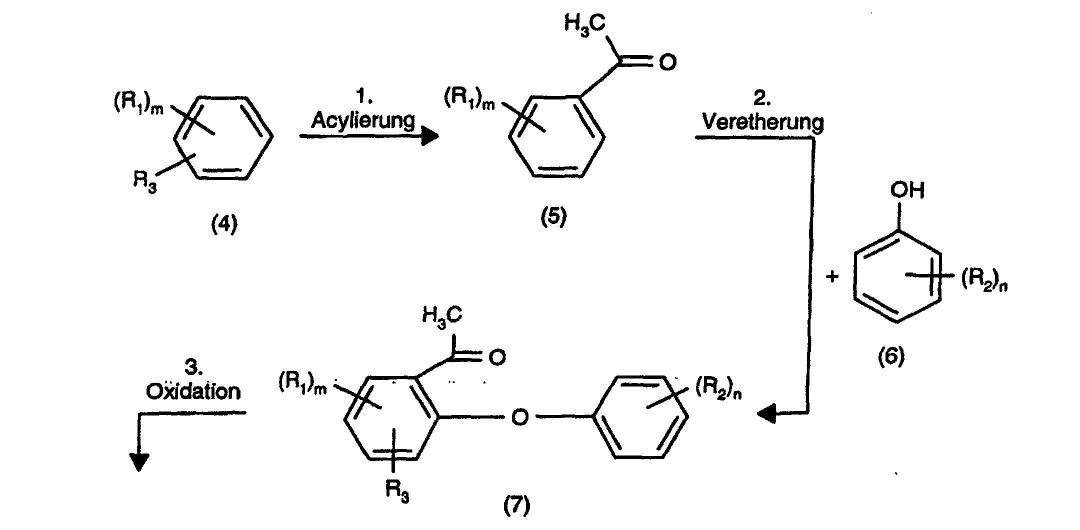 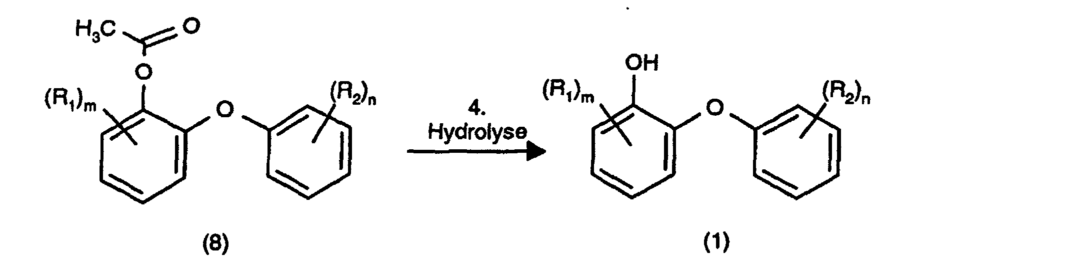 dadurch gekennzeichnet, dass die zweite Stufe in Anwesenheit einer starken organischen oder anorganischen Base, eines Kupferkatalysators und eines inerten organischen Lösungsmittels, ausgewählt aus Toluol, und Xylol-Isomerengemischen, durchgeführt wird,

Im obigen Schema haben R₁, R₂, m und n die in Formel (1) angegebene Bedeutung.

Im ersten Reaktionsschritt (Acylierungsreaktion) werden Verbindungen der Formel (5) hergestellt. Gewöhnlich wird diese Reaktion in Anwesenheit einer Lewis-Säure, wie z.B. Aluminiumhalogenid, insbesondere Aluminiumchlorid durchgeführt. Die Lewis-Säure wird dabei in 1 bis 3, vorzugsweise 1,25 bis 2 molarer Menge, bezogen auf die halogenierte Verbindung der Formel (5), eingesetzt. Als Acylierungsreagenz kommt für diese Reaktion ein Acylhalogenid, insbesondere Acetylchlorid, in Frage. Lewis-Säure und Acylierungsreagenz werden dabei vorzugsweise in äquimolaren Mengen eingesetzt. Die Reaktion wird in den für Friedel-Crafts-Reaktionen üblichen Lösungsmitteln durchgeführt, wie z.B. Methylenchlorid oder Ethylenchlorid. Die Reaktionszeit spielt für diese Reaktionsstufe eine untergeordnete Rolle und kann in einem breiten Rahmen, von z.B. 1 bis 18 Stunden variieren.

In der zweiten Reaktionsstufe werden die Verbindungen der Formel (7) hergestellt. Die Veretherung der freien OH-Gruppe der halogenierten Phenolverbindung der Formel (6) erfolgt gewöhnlich in alkalischem Medium unter Verwendung einer starken organischen oder vorzugsweise anorganischen Base, wie z.B. NaOH oder KOH und in Anwesenheit eines Kupferkatalysators und eines inerten organischen Lösungsmittels, wie z.B. Toluol oder eines Xylol-lsomeren-Gemisches. Die Reaktionszeiten für diesen Reaktionsschritt betragen dabei gewöhnlich 1 bis 24, vorzugsweise 2 bis 10 Stunden, die Temperatur reicht von 80 bis 250, vorzugsweise 100 bis 170°C.

In der dritten Reaktionsstufe (Oxidation) werden Verbindungen der Formel (8) hergestellt. Die Oxidation der Acylverbindung der Formel (7) zur Verbindung der Formel (8) (Baeyer-Villiger-Oxidation) lässt sich mit verschiedenen Oxidationsmitteln durchführen. Geeignete Oxidationsmittel sind z.B.:
- Gemisch aus verdünnter Peressigsäure und Acetanhydrid in Gegenwart einer katalytischen Menge Perchlorsäure;
- m-Chlorperbenzoesäure(MCPBA) in Wasser;
- Di-peroxydodecandisäure (DPDDA);
- Gemisch aus verdünnter Peressigsäure und Acetanhydrid und Schwefelsäure;
- Perbenzoesäure (PBA)
- Gemisch aus Natriumborat und Trifluoressigsäure;
- Gemisch aus Ameisensäure, Wasserstoffperoxid, Acetanhydrid, Phosphorpentoxid und Essigsäure;
- Gemisch aus Essigsäure, Wasserstoffperoxid, Acetanhydrid und Phosphorpentoxid;
- Gemisch aus K₂S₂O₈, Schwefelsäure und einem 1:1-Wasser/Methanol-Gemisch;
- Gemisch aus Essigsäure und dem Kaliumsalz der Monoperoxomaleinsäure;
- Gemisch aus Trichlormethylen, dem Kaliumsalz der Monoperoxomaleinsäure und Natriumhydrogensulfat;
- Gemisch aus Maleinsäureanhydrid, Acetanhydrid, Wasserstoffperoxid und Trichlormethan;
- Gemisch aus Maleinsäureanhydrid, einem Hamstoff-Wasserstoffperoxid-Komplex und Essigsäure;
- Mg-mono-perphthalat;
- Gemisch aus Essisäureanhydrid, Schwefelsäure und H₂O₂;
- Gemisch aus Dichloressigsäure und H₂O₂.

Vorzugsweise wird für die Oxidation m-Chlorperbenzoesäure (MCPBA), ein Gemisch aus Natriumborat und Trifluoressigsäure oder ein Gemisch aus Essisäureanhydrid und H₂O₂ verwendet. Gegebenenfalls kann dem Oxidationsmittel noch ein handelsübliches Netzmittel zugesetzt werden. Die Reaktionszeiten liegen in einem breiten Bereich und reichen von ca. 0,5 bis ca. 15, vorzugsweise 1 bis 8 Stunden. Die Reaktionstemperatur reicht von- 20 bis ca. 100°C, vorzugsweise von 0 bis ca. 85°C.

Die anschliessende Hydrolyse zum gewünschten Halogen-hydroxydiphenylether der Formel (1) verläuft im sauren oder alkalischen Medium quantitativ.

Ganz besonders bevorzugte Verbindungen der Formel (1) entsprechen der Formel 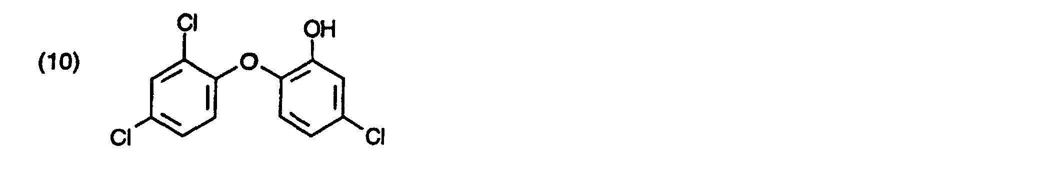 oder der Formel

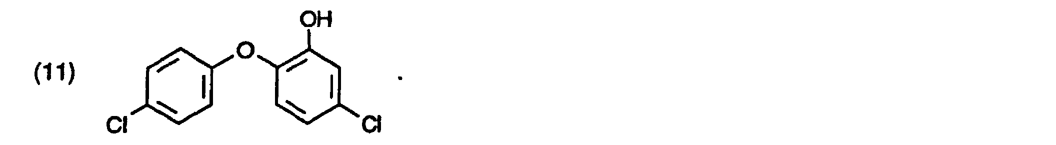

Die erfindungsgemäss hergestellten halogenierten Hydroxy-Diphenylverbindungen sind in Wasser unlöslich, dagegen in verdünnter Natrium- und Kaliumhydroxid-Lösung und in praktisch allen organischen Lösungsmitteln löslich. Dank dieser Löslichkeitsvoraussetzungen ist ihre Anwendbarkeit zur Bekämpfung von Mikroorganismen, insbesondere von Bakterien, und als Desinfektionsmittel zum Schützen von organischen Materialien und Gegenständen vor dem Befall von Mikroorganismen sehr vielseitig. So kann man sie z.B. zusammen mit Netz- oder Dispergiermitteln z.B. als Seifen- oder Syndetlösungen zur Desinfektion und Reinigung der menschlichen Haut und Hände, harter Gegenstände sowie in Zahnpflegemitteln in verdünnter oder unverdünnter Form auf diese auftragen.

Die folgenden Beispiele erläutern die Erfindung, ohne sie darauf zu beschränken.

### Herstellungsbeispiele

### Beispiel 1: Herstellung von 2,5-Dichloracetophenon (Erste Reaktionsstufe)

### Reaktionsschema:

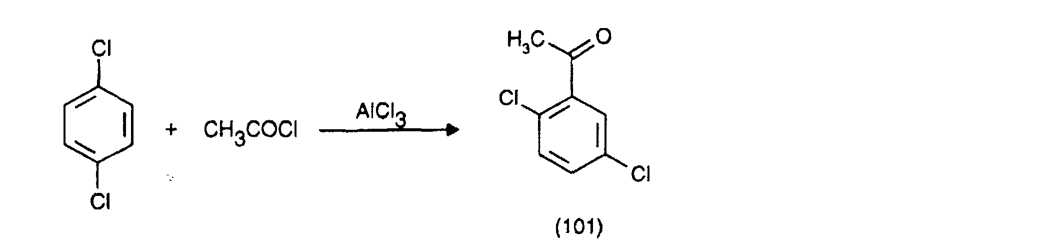

In einer Apparatur mit aufgesetztem Tropftrichter, Rührer und Rückflusskühler werden 147 g (1,0 mol) p-Dichlorbenzol bei 60°C vollständig geschmolzen. Zu der Schmelze werden 120 g (0,9 mol) wasserfreies AlCl₃ gegeben. Anschliessend tropft man zu der gut rührbaren Suspension bei 60°C innerhalb ca. 1 Stunde 39,3 g (0,5 mol) Acetylchlorid zu, wobei langsam eine klare Lösung entsteht. Nach Aufheizen auf 110°C wird 7 Stunden bei dieser Temperatur gerührt. Nach dem Abkühlen auf Raumtemperatur wird die braune Reaktionsmasse durch vorsichtiges Abgiessen auf eine Mischung aus 200 ml Wasser und 200 g Eis hydrolysiert. Die Temperatur der Mischung wird während der Hydrolyse durch Aussenkühlung zwischen 30 und 40°C gehalten. Nach Trennung der Phasen wird die untere, organische Phase mit 400 ml Wasser gewaschen und nach erneuter Phasentrennung einer fraktionierenden Destillation unterzogen. Die wässrigen Phasen werden verworfen.

Ausbeute: 66 g 2,5-Dichloracetophenon ( 70 % d.Th., bezogen auf Acetylchlorid)

### Beispiel 2: Herstellung von 1-(5-Chloro-2-(2,4-dichlorophenoxy)-phenyl-ethanon (zweite Reaktionsstufe)

### Reaktionsschema:

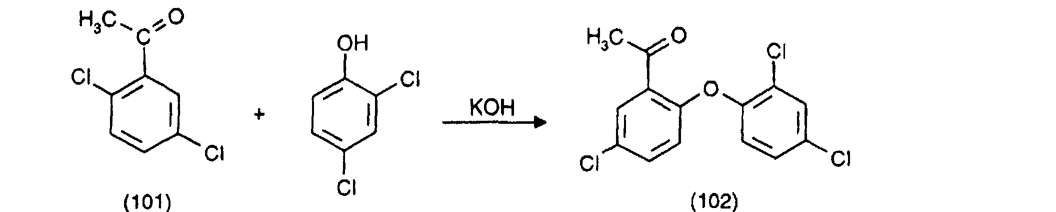

163 g 2,4-Dichlorphenol werden zusammen mit 22,0 g 85%iger KOH und 167 ml Xylol-Isomerengemisch vorgelegt. Die Mischung wird auf Rückfluss erhitzt und das Wasser azeotrop abdestilliert. Die rotbraune, klare Lösung wird dann auf 100°C gekühlt, mit 189 g 2,5-Dichloracetophenon (Verbindung der Formel (101)) und 0,8 g basischem Kupfercarbonat versetzt, auf 140°C erwärmt und bei dieser Temperatur während 8 Stunden gerührt. Dabei färbt sich die Reaktionslösung dunkel rotbraun und es fällt ein weisser Niederschlag aus, der abfiltriert wird. Nach dem Abdestillieren des grössten Teils des Xylols in leichtem Wasserstrahlvakuum erhält man eine Fraktion von 208 g, die neben den Ausgangsprodukten 2,4-Dichlorphenol und 2,5-Dichloracetophenon noch etwas Xylol enthält (Sdp. 30-85°C/0,5-1 mgfm Hg). Es werden 82 g einer zweiten, leicht gelblichen Fraktion (Sdp. 165 -175°C/1 mm Hg) der Verbindung der Formel (102) erhalten, entsprechend einer Ausbeute von 82% d.Th., bezogen auf eingesetztes KOH (Fp. = 91°C).

| Analyse für C₁₄H₉Cl₃O₂(MG = 315,58): | | | | |
|---|---|---|---|---|
| | C | H | Cl | Q |
| Berechnet [%] | 53,28 | 2,87% | 33,7% | 10,14 |
| Gefunden [%] | 53,31 | 2,85 | 33,37 | 10,31 |

### Beispiel 3: Herstellung von 1-(5-Chloro-2-(4-chlorophenoxyl-phenyl)-ethanon (2. Reaktions

### stufe)

Man verfährt wie in Beispiel 2 beschrieben, jedoch unter Verwendung von 128,6 g 4-Chlorphenol anstelle von 163 g Dichlorphenol.

Nach einer Reaktionszeit von 2 Stunden bei 140°C und der anschliessenden Aufarbeitung wie in Beispiel 2 beschrieben, erhält man nach einer Vorfraktion von 223 g (Sdp. 30-112°C/1 mm Hg) eine Hauptfraktion von 91 g (Sdp. 112°C-173°C/1 mm Hg), die neben dem Ausgangsprodukt 2,5-Dichloracetophenon mehr als 80% des Reaktionsproduktes der Formel 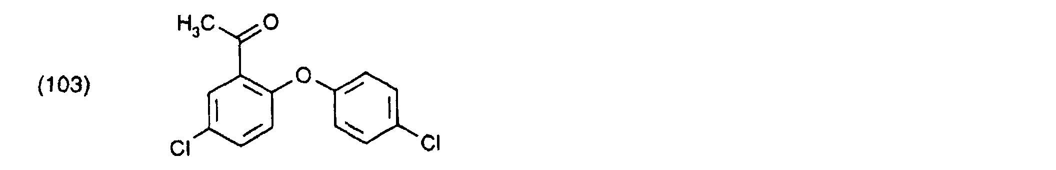 enthält, das beim Abkühlen zu einem weissen Pulver erstarrt (Fp.= 64°C).

| Analyse für C₁₄H₁₀Cl₂O₂ (MG = 281,14): | | | | |
|---|---|---|---|---|
| | C | H | Cl | O |
| Berechnet [%] | 59,81 | 3,59 | 25,22 | 11,38 |
| Gefunden [%] | 59,74 | 3,48 | 25,49 | 11,29 |

### Beispiel 4: Herstellung von 5-Chloro-2-(2,4-dichlorophenoxy)-phenol durch Baeyer-Villiger-Oxidation mit m-Chlorperbenzoesäure (MCPBA) (dritte und vierte Reaktionsstufe)

### Reaktionsschema:

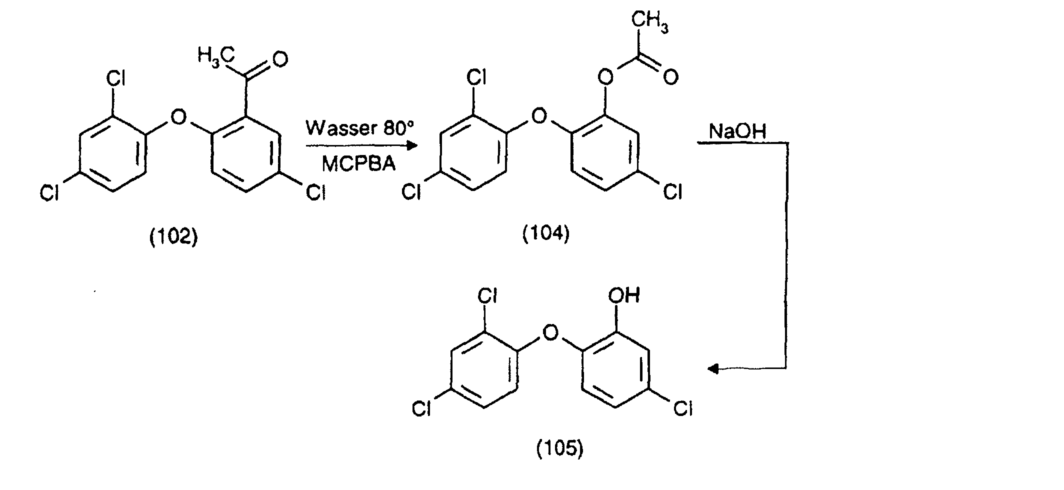

6,3 g der Acetylverbindung der Formel (102) werden in 40 ml deionisiertem Wasser bei 20°C angeschlämmt. Man streut 9,8 g m-Chlor-perbenzoesäure (MCPBA) zu und erwärmt das Gemisch unter Rühren. Ab 52°C bildet sich eine Harz-/Wasserphase, man heizt bis ca. 80°C und hält diese Temperatur während 6 Stunden.

Zur Vernichtung des überschüssigen Peroxids versetzt man mit 0,5 g Natriumhydrogensulfit. Durch Zugabe von 50 ml Ethylenchlorid und 4 g 10N NaOH erhält man zwei klare Phasen. Die Wasserphase mit einem pH-Wert von ca. 12 wird abgetrennt, die Lösungsmittelphase mit Wasser neutral gewaschen. Nach Destillation des Lösungsmittels verbleiben 5,5 g kristallisierende Verbindung der Formel (104) (Phenolester-Zwischenprodukt).

Zur Hydrolyse löst man den Phenolester in 50 ml Ethylenchlorid und 10 ml 5N Natronlauge. Man heizt auf 70-73°C, hält diese Temperatur während 15 Minuten, stellt dann den pH-Wert mit Essigsäure auf ca. 4 und trennt die Phasen. Nach Entfernung des Lösungsmittels erhält man 4,9 g beigefarbenes Rohprodukt der Formel (105).

Nach Klärfiltration und Umkristallisation aus Petrolether 80/110 erhält man das reine Produkt in farblosen Kristallen und mit einem Schmelzpunkt von 56 bis 57°C.

### Beispiel 5: Herstellung von 5 -Chloro -2-(2,4-dichlorophenoxy)-phenol durch Baeyer-Villiger-Oxidation mit NaBO₃ (dritte und vierte Reaktionsstufe)

6,3 g der Acetylverbindung der Formel (102) werden in 20 ml Trifluoressigsäure suspendiert und bei 20°C mit 9,3 g Natriumperborat-Tetrahydrat versetzt. Man erwärmt auf 40°C und hält diese Temperatur während 90 Minuten unter gutem Rühren. Nach Hydrolyse des gebildeten Phenolesters und Aufarbeitung analog Beispiel 4 erhält man 5,4 g Rohprodukt der Formel (105).

### Beispiel 6: Herstellung von 5-Chloro-2-(2,4-dichlorophenoxy)-phenol durch Baeyer-Villiger-Oxidation mit Essigsäureanhydrid/H₂O₂

18 ml Essigsäure-Anhydrid werden bei -5°C mit 4,5 ml 98%-iger Schwefelsäure gemischt. Dazu tropft man unter sehr starkem Rühren bei -5 bis -3°C in 25 Minuten 4,2 ml 30%-iges Wasserstoffperoxid. Die milchige Emulsion wird bei -5°C mit 12,5 ml Methylenchlorid versetzt, wobei sich eine klare Lösung bildet. Diese gibt man nun unter sehr starkem Rühren in 3 Minuten zu einer Vorlage von 7,9 g der Acetylverbindung der Formel (102), 15 ml Methylenchlorid, 18 ml Essigsäure 100% und 13,5 ml Schwefelsäure 98%, bei einer Temperatur 0 bis -5°C.

Das Reaktionsgemisch ist zweiphasig und dunkel. Man hält die Reaktion während einer Stunde bei 0 bis 5°C, dann 4 Stunden bei 10°C und 1 Stunde bei 15°C. Man lässt abschliessend während 20 Stunden bei 20°C ausreagieren, wobei auch der intermediär gebildete Phenolester der Formel (104) bereits teilweise zum Phenolderivat der Formel (105) hydrolysiert. Nach Zerstörung des überschüssigen Wasserstoffperoxyd wird das Methylenchlorid ausdestilliert. Zur vollständigen Hydrolyse hält man die Temperatur während 4 Stunden bei 100°C. Das Produkt wird via Methylenchlorid-Extraktion gewonnen. Nach Destillation des Lösungsmittels verbleibt ein öliger Rückstand von 7g des rohen Produktes der Formel (105), welcher nach üblicher Reinigung das Produkt mit einem Schmelzpunkt von 56 bis 57°C liefert.

### Beispiel 7: Herstellung von 5-Chloro-2-(4-chloro-phenoxy)-phenol (dritte und vierte Reaktionsstufe)

### Reaktionsschema:

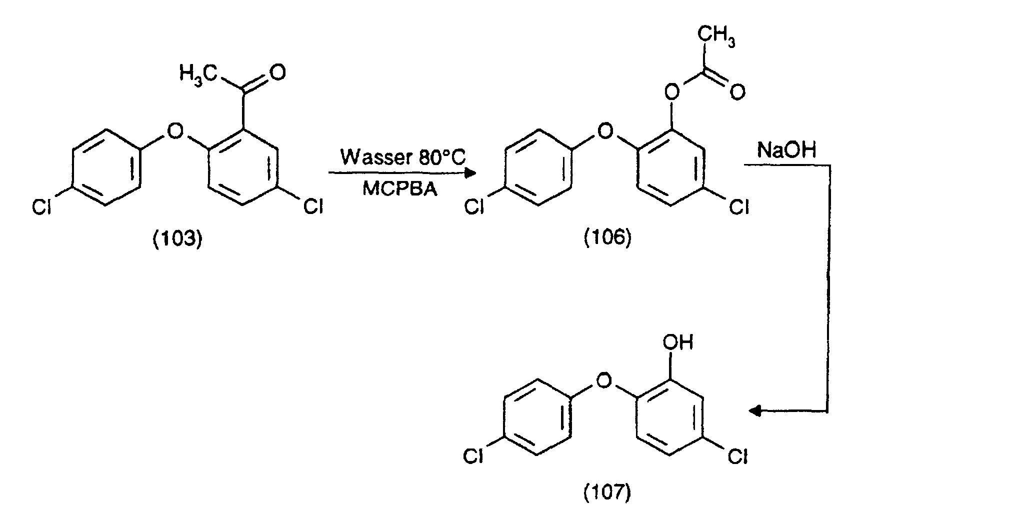

14 g der Acetylverbindung der Formel (103) werden mit einem Netzmittel in 100 ml deionisiertem Wasser bei 20°C angeschlämmt. Man streut 29 g 70%ige 3-Chlor-perbenzoesäure (MCPBA) zu und erwärmt das Gemisch unter Rühren. Ab 52°C bildet sich eine Harz-/Wasserphase, man heizt bis ca. 80°C und hält diese Temperatur während 7 Stunden.

Zur Vernichtung des überschüssigen Peroxids versetzt man mit 0,5 g Natriumhydrogensulfit. Durch Zugabe von 50 ml Xylol-lsomerengemisch und 9 g NaOH 10N erhält man zwei klare Phasen. Die Wasserphase mit einem pH-Wert von ca. 12 wird abgetrennt, die die Verbindung der Formel (106) enthaltende Lösungsmittelphase mit Wasser neutral gewaschen.

Zur Hydrolyse des Esters wird die Xylolphase mit 24 g NaOH 10% versetzt und 5 Stunden unter Rückfluss (ca. 95°C) gerührt. Nun wird die Xylolphase abgetrennt und die hellbraune Wasserphase bei 25°C mit 4 g 34%iger Salzsäure auf einen pH-Wert von ca. 3 gestellt. Dabei fällt das Produkt in sandiger, beigefarbener Form aus und kann nach Filtrieren auf der Nutsche mit Wasser gründlich gewaschen werden. Nach dem Trocknen erhält man 5 g des Rohproduktes der Formel (107) mit einem Schmelzpunkt von 73 bis 74°C.

Nach Umkristallisation aus Petrolether 80/110 wird die Reinsubstanz in farblosen Kristallen mit einem Schmelzpunkt von 74 bis 74,5°C erhalten.

### Beispiele 8 bis 14 : Analog dem oben beschriebenen Herstellungsverfahren sind folgende Verbindungen erhältlich :

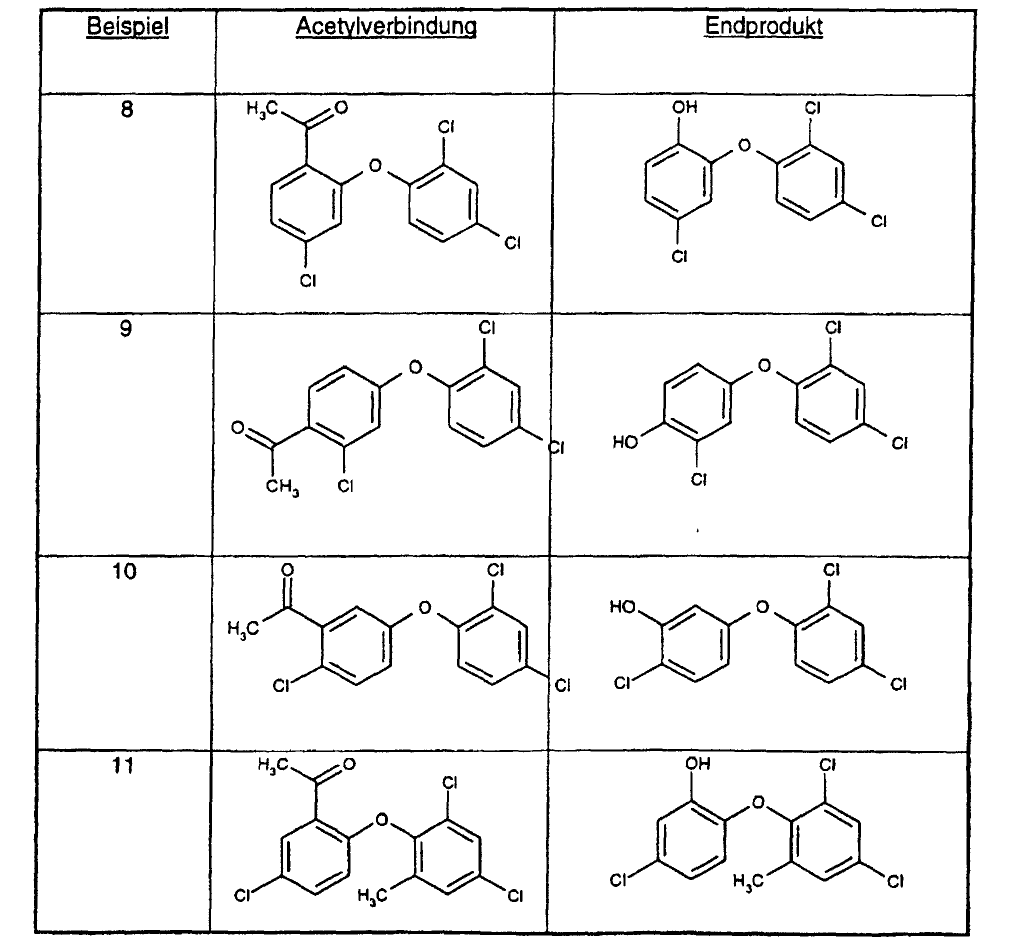

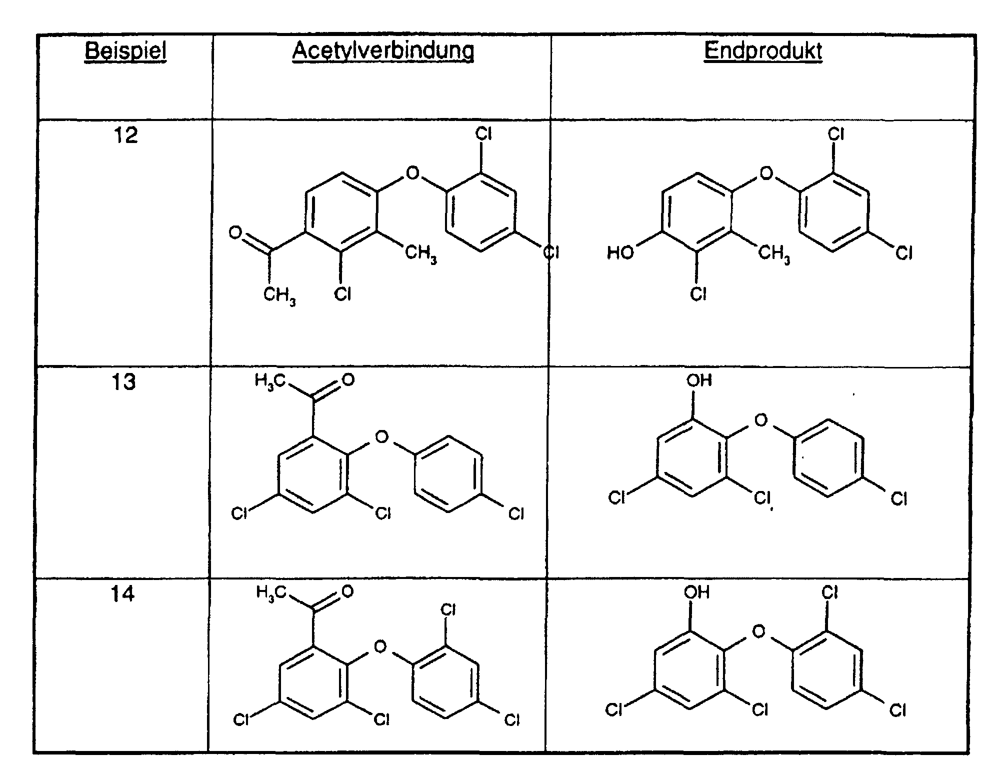

## Patentansprüche

1. Verfahren zur Herstellung von halogenierten Hydroxydiphenylverbindungen der Formel 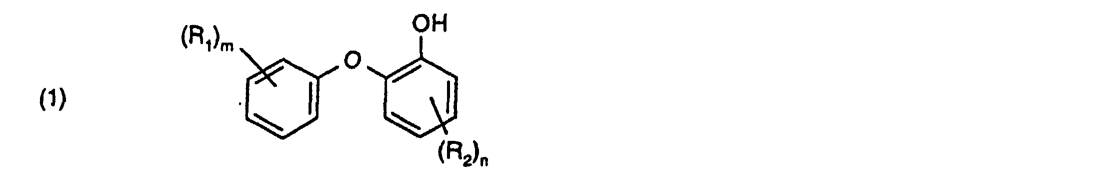 durch Acylierung einer halogenierten Benzolverbindung (erste Stufe), Veretherung der acylierten Verbindung mit einer halogenierten Phenolverbindung (zweite Stufe), Oxidation der veretherten Verbindung (dritte Stufe) und Hydrolyse der oxidierten Verbindung in einer vierten Stufe entsprechend dem folgenden Reaktionsschema: 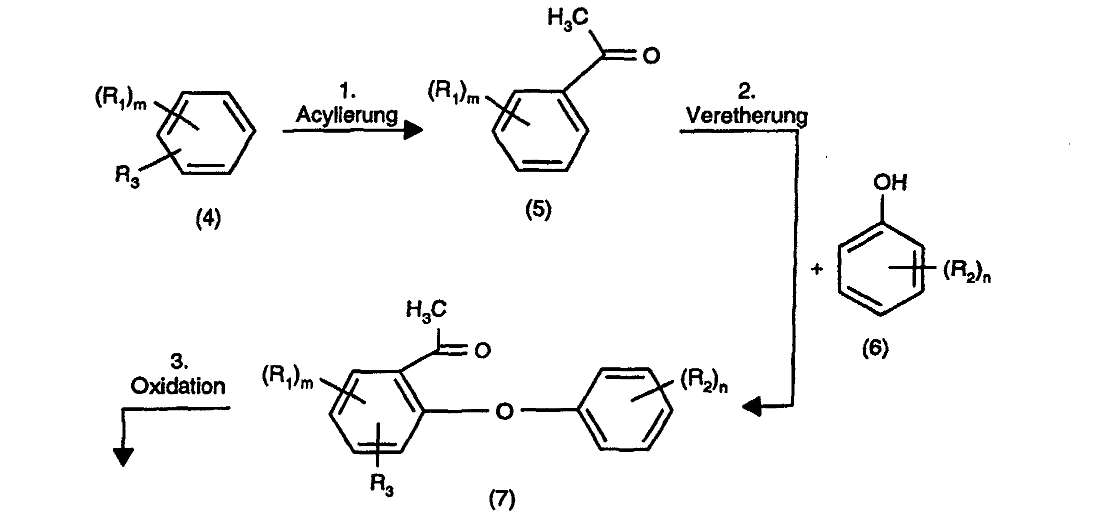 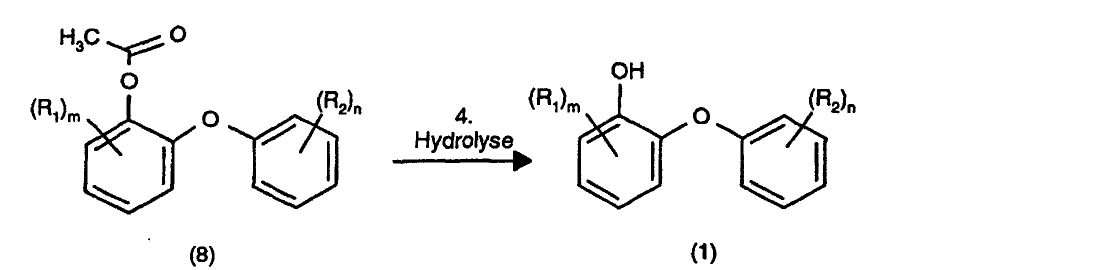 **dadurch gekennzeichnet, dass** die zweite Stufe in Ansesenheit einer starken organischen oder anorganischen Base, eines Kupferkatalysators und eines inerten organischen Lösungsmittels, ausgewählt aus Toluol, und Xylol-Isomerengemischen, durchgeführt wird,
wobei
R₁ und R₂ Cl ;
m 2; und
n 1 oder;
m und n 1;
bedeuten.

2. Verfahren nach Anspruch, **dadurch gekennzeichnet, dass** die Acylierungsreaktion (erste Stufe) in Anwesenheit einer Lewis-Säure durchgeführt wird.

3. Verfahren nach einem der Ansprüche 1 oder 2, **dadurch gekennzeichnet, dass** für die Acylierungsreaktion ein Acylhalogenid, vorzugsweise Acetylchlorid verwendet wird.

4. Verfahren nach einem der Ansprüche 1 bis 3, **dadurch gekennzeichnet, dass** die Oxidation in Anwesenheit von m-Chlorperbenzoesäure durchgeführt wird.

5. Verfahren nach einem der Ansprüche 1 bis 3, **dadurch gekennzeichnet, dass** die Oxidation in Anwesenheit eines Gemisches aus Natriumborat und Trifluoressigsäure durchgeführt wird.

6. Verfahren nach einem der Ansprüche 1 bis 5, **dadurch gekennzeichnet, dass** es die Herstellung der Verbindung der Formel 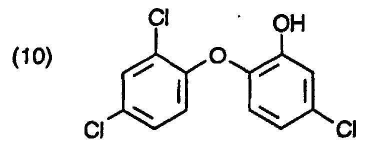 betrifft.

7. Verfahren nach einem der Ansprüche 1 bis 5, **dadurch gekennzeichnet, dass** es die Herstellung der Verbindung der Formel 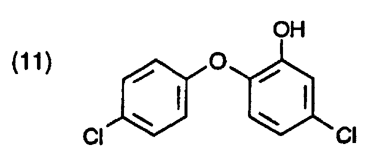 betrifft.

## Claims

1. A process for the preparation of halogenated hydroxydiphenyl compounds of the formula 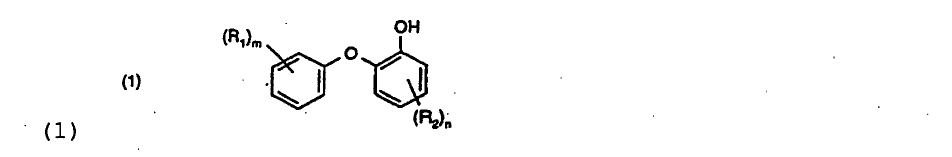 by acylation of a halogenated benzene compound (first stage), etherification of the acylated compound with a halogenated phenol compound (second stage), oxidation of the etherified compound (third stage) and hydrolysis of the oxidized compound in a fourth stage, according to the following reaction scheme: 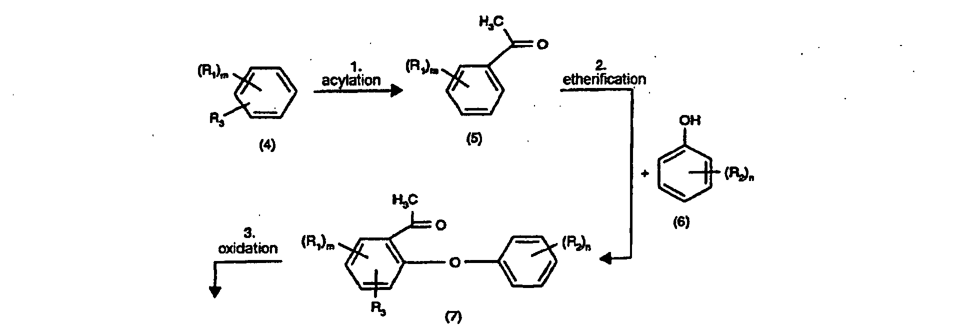 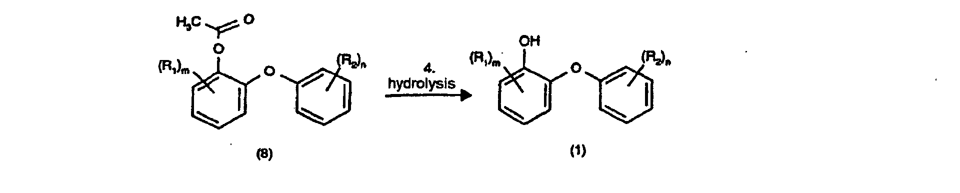 **characterized in that** the second stage is carried out in the presence of a strong organic or inorganic base, a copper catalyst and an inert organic solvent selected from the group consisting of tolvene and xylene isomer mixtures,
where
R₁ and R₂ are Cl;
m is 2; and
n is 1; or
m and n are 1.

2. A process according to claim 1 **characterized in that** the acylation reaction (first stage) is carried out in the presence of a Lewis acid.

3. A process according to either one of claims 1 and 2, **characterized in that** an acyl halide, preferably acetyl chloride, is used for the acylation reaction.

4. A process according to any one of claims 1 to 3, **characterized in that** the oxidation is carried out in the presence of m-chloroperbenzoic acid.

5. A process according to any one of claims 1 to 3, **characterized in that** the oxidation is carried out in the presence of a mixture of sodium borate and trifluoroacetic acid.

6. A process according to any one of claims 1 to 5, **characterized in that** it relates to the preparation of the compound of the formula

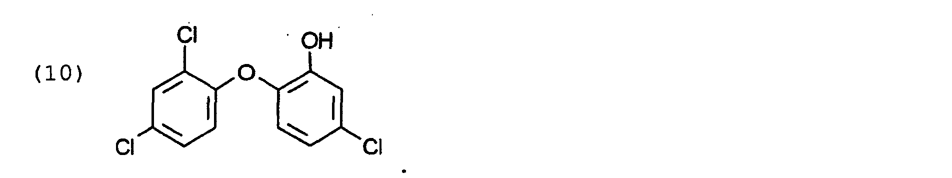

7. A process according to any one of claims 1 to 5, which relates to the preparation of the compound of the formula

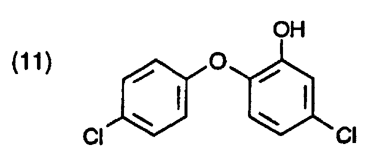

## Revendications

1. Procédé pour la préparation de composés hydroxydiphényliques halogénés de formule 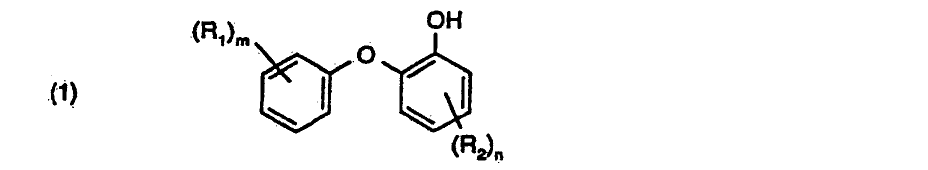 par acylation d'un composé benzénique halogéné (première étape), éthérification du composé acylé par un composé phénolique halogéné (deuxième étape), oxydation du composé éthérifié (troisième étape) et hydrolyse du composé oxydé dans une quatrième étape conformément au schéma réactionnel suivant 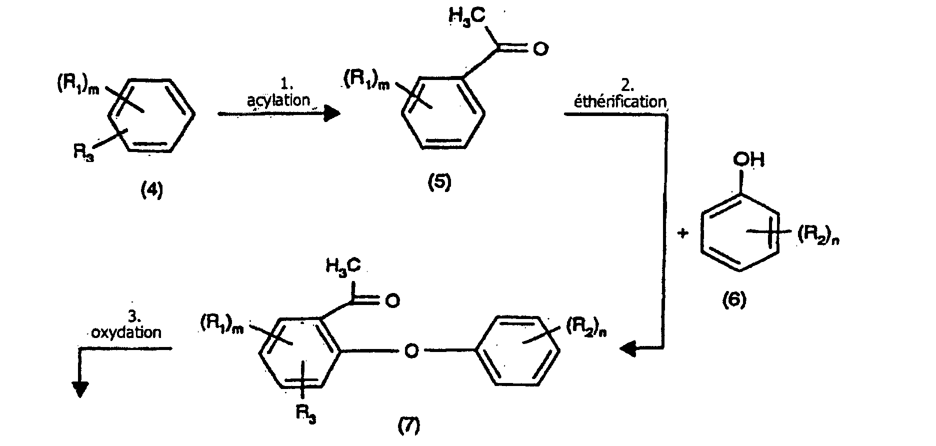 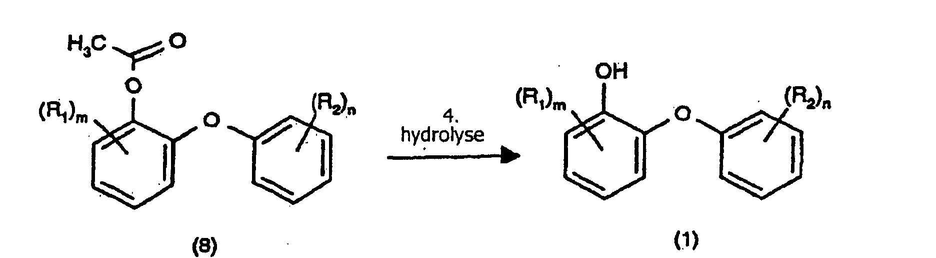 **caractérisé en ce que** la deuxième étape est mise en oeuvre en présence d'une base organique ou inorganique forte, d'un catalyseur au cuivre et d'un solvant inerte, pris parmi le toluène et des mélanges de xylènes isomères,
où
R₁ et R₂ représentent Cl;
m vaut 2 ; et
n vaut 1, ou
m et n valent 1.

2. Procédé selon la revendication 1, **caractérisé en ce qu'**on met en oeuvre la réaction d'acylation (première étape) en présence d'un acide de Lewis.

3. Procédé selon l'une des revendications 1 ou 2, **caractérisé en ce qu'**on utilise pour la réaction d'acylation un halogénure d'acyle, de préférence le chlorure d'acétyle.

4. Procédé selon l'une des revendications 1 à 3, **caractérisé en ce qu'**on met en oeuvre l'oxydation en présence d'acide m-chloroperbenzoïque.

5. Procédé selon l'une des revendications 1 à 3, **caractérisé en ce qu'**on met en oeuvre l'oxydation en présence d'un mélange de borate de sodium et d'acide trifluoracétique.

6. Procédé selon l'une des revendications 1 à 5, **caractérisé en ce qu'**il concerne la préparation du composé de formule

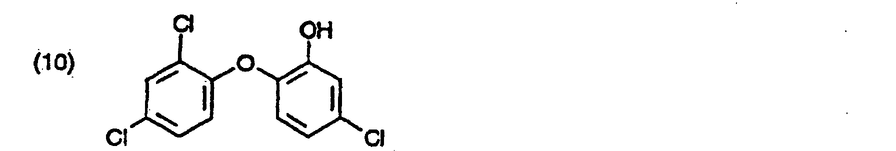

7. Procédé selon l'une des revendications 1 à 5, **caractérisé en ce qu'**il concerne la préparation du composé de formule
